# EUROPEAN PATENT SPECIFICATION

(11) **EP 3 052 054 B1**
(45) Date of publication and mention of the grant of the patent: **13.11.2019**
(21) Application number: 14789533.8
(22) Date of filing: 30.09.2014
(51) Int. Cl.: A61F 2/44

(54) **SPINAL IMPLANT FOR INTERBODY USE**
WIRBELSÄULENIMPLANTAT ZUR INTERVERTEBRALEN VERWENDUNG
IMPLANT SPINAL POUR UTILISATION INTERCORPS

(30) Priority: 02.10.2013 WO PCT/EP2013/070589
(43) Date of publication of application: 10.08.2016
(73) Proprietor: Motion Medical, 3390 Houwaart (BE)
(72) Inventor: MESDOM, Steven, 3390 Hauwaart (BE); SIAU, Vincent, 3390 Hauwaart (BE)
(74) Representative: Gevers Patents
(86) International application number: PCT/EP2014/070902
(87) International publication number: WO 2015/049222

(56) References cited:
- WO-A1-2004/008983
- US-A- 4 865 603
- US-A1- 2002 156 529
- US-A1- 2008 294 260
- US-A1- 2009 276 053
- US-A1- 2010 168 798
- US-A1- 2010 262 244
- US-A1- 2012 158 144
- US-A1- 2012 316 650

## Description

The current invention relates to a spinal implant according to the preamble of the first claim, for example aiming to replace damaged spinal discs in between two vertebrae.

Such spinal implants are already known to the person skilled in the art. US2012/0312778 A1 for example describes an interbody spinal implant. The spinal implant comprises a top surface, a bottom surface, opposing lateral sides, opposing anterior and posterior portions, a substantially hollow center, and a single vertical aperture. At least one of the aforementioned surfaces forms a roughened surface topography having a regular repeating pattern. The implant comprises a substrate, more in particular a body of the implant, and, on top of the body, an integration layer, more in particular an integration plate, provided with the roughended surface topography. According to certain embodiments, the integration plate is made of titanium as titanium is a metal that shows good cell adhesion properties which is a desired property for body implants as they allow an improved healing effect. According to further embodiments described in US2012/0312778 A1, the body of the implant is made of polyetheretherketone (PEEK), a plastic material which is frequently used in body implants. PEEK is a highly inert material leading to weaker cell adhesion. The polymer material is covered with the titanium integration plate provided with the roughened surface topography. The roughened topography of the integration layer is obtained by using a subtractive or additive process directly on the titanium integration plate.

However, such implants suffer drawbacks. Titanium is a very hard material with a greater stiffness (modulus of elasticity) than bone: this can result in the implants becoming embedded in the endplates of the vertebrae, which may cause considerable pain for the patient. The combination of the PEEK body with the titanium integration plate offsets a bit the disadvantages of titanium's stiffness but still has a relatively large risk of damaging adjacent vertebrae. US 2008/294260 A1 discloses the preamble of claim 1.

Therefore, it is an aim to provide a spinal implant that has a decreased risk of damaging adjacent vertebrae and at the same time benefit from titanium's cell-growth promoting characteristics.

This is achieved according to the present invention by a spinal implant showing the technical features of the characterising portion of the first claim.

Thereto, the integration layer of the spinal implant is in the form of a coating applied onto the substrate.

It has been found that such a coating layer on the substrate can greatly have a greatly decreased thickness. Thus, the implant will show more the better elasticity and/or stiffness properties of the polymer substrate decreasing the risk that vertebrae become damaged, especially when a metal containing coating is used. On the other hand, the presence of the coating can provide improved cell adhesion properties such that an improved healing effect is obtained. In other words, such a configuration allows conferring the more adapted mechanical properties of, for example, PEEK, thus for example reducing the risk of embedment in the endplates of the vertebrae, together with the improved cell-growth promotion characteristics of the coating. This combination allows, for example, an acceleration of the development of a stable bone bridge and a faster healing of the patient.

It has also been found that by applying a coating on the substrate, the thickness of the integration layer can be reduced down to the nanometer range. This step of coating can be for example performed by using a sputtering deposition technique. In this process, for example a high-energy ionic beam (for example an argon beam) is shot onto a titanium wafer, and the beam releases titanium atoms which are then deposited on the object to be coated. Using this process, it is possible to produce controlled thicknesses for example with a precision in the nanometer range, thus allowing relatively thin coatings. When using coating techniques, it therefore becomes possible to apply the texture, in which the distance, preferably depth, between adjacent local extrema is less than 1 mm, for example in micrometer range, into and/or onto the biocompatible polymer material, for example directly into the biocompatible polymer material, and to subsequently cover the texture in the polymer material with the coating without substantially altering the texture. Without wanting to be bound by any theory, it is believed that this is due because of the relatively large difference in between the distance, preferably depth, between the adjacent local extrema and the thickness of the coating. Therefore, the texture of the implant is substantially defined by the texture of the substrate.

According to preferred embodiments of the current invention, the coating is a metal containing coating. It has been found that such coating can be relatively easily applied by sputtering techniques and can offer improved cell adhesion properties.

According to more preferred embodiments of the current invention, the metal of the metal containing coating is one of the list consisting of titanium, aluminium, vanadium, tantalum, stainless steel, and alloys of these materials. It has been found that these metals, especially titanium, have very good cell-growth promoting characteristics, and improve healing. More preferably, the metal containing coating is a metal coating.

According to preferred embodiments of the current invention, the metal containing coating comprises a metal coating.

According to more preferred embodiments of the current invention, the metal of the metal coating is one of titanium and a titanium alloy. Preferably, the metal coating is a titanium coating. It has been found that titanium has good cell adhesion properties and therefore offers good healing properties.

According to preferred embodiments of the current invention, the metal containing coating comprises a metal oxide, the metal of the metal oxide being one of the list consisting of titanium, aluminium, vanadium, tantalum, stainless steel, and alloys of these materials, for example titanium oxide.

According to an example, the coating comprises any one or more of calcium phosphate, hydroxyapatite. It has been found that such materials also offer good cell adhesion properties.

According to preferred embodiments of the current invention, the coating is in the form of any one of amorphous, crystalline, polycrystalline, nanocrystalline, etc.

It has further been found that such coatings, especially titanium comprising coatings, for example a titanium coating, have a relatively small risk of flaking off the substrate, for example a PEEK substrate, as it has been found that coatings, especially coatings having a thickness of between 0.3 nm - 5 µm, preferably between 100 nm - 2,5 µm, more preferably 250 nm, follow the deformation of the substrate, also adding to the improved elasticity and/or stiffness properties. Such properties for example avoid that the coating flakes off the substrate. Especially when using visible coatings, such as titanium coating, it has been found that a coating of 250 nm offers a visual check that a coating is present in the implant, for example for the practitioner, for example a surgeon, having to perform the operation.

Moreover, the reduced thickness of the coating layer, for example the thicknesses mentioned above, on the substrate also improves the transparency of the spinal implant, especially when the coating comprises a metal containing coating, for example for X-rays, for example in classic X-ray photography and/or X-ray computed tomography (CT), magnetic resonance imaging (MRI) and/or single-photon emission computed tomography (SPECT), so that the implant is less visible on X-ray images so that the surrounding body tissue, for example bone tissue, is better visible. Therefore, for example when monitoring the course of healing, for example the formation of bone bridges as well as for example the specific position of the implant can be more clearly and more distinctly observed and recognized.

According to preferred embodiments of the current invention, the texture is engineered in the form of grooves. It has been found that such a texture offers good cell adhesion properties. Preferably, the grooves and/or holes follow a periodic pattern. For example, a periodic pattern of grooves, preferably a periodic pattern of parallel grooves, wherein the grooves have a depth of between 200 nm and 1 µm or higher, for example 200nm and 2 µm or even 500nm - 1500 nm, preferably a depth of between 1,0 µm and 2,0 µm, and a width of between 1 µm and 20 µm, or even between 1µm and 10 µm, for example a depth of about 500 nm and a width of about 4 µm. According to preferred embodiments, a depth of between 0,5 µm and 1,5 µm, more preferably 1,0 µm and 1,5 µm, and for example a width of about 7 µm is preferred. It has been found that surface roughness and/or waviness can improve the cell adhesion properties of the surface of the implant. It has for example been found that grooves allow increasing the cell adhesion properties as they induce cell contact guidance.

According to preferred embodiments of the current invention, the texture is engineered in the form of grooves. According to further preferred embodiments of the current invention, the grooves comprise at least a first group of substantially parallel grooves. According to other embodiments of the current invention, the grooves comprise at least a first and a second group of substantially parallel grooves, the grooves of the two groups crossing each other, forming a grid in the form of an array of quadrilaterals.

According to preferred embodiments of the current invention, the integration layer is located on the outer surface of the substrate, allowing a more direct contact between the integration layer and the human body cells and thus optimizing the integration of the implant in the human body and therefore, the healing.

According to preferred embodiments of the current invention, the distance between the adjacent local extrema is between 0,3 µm and 150 µm, preferably 0,5 µm and 130 µm. It has been found that such distances confer a roughened texture to the integration layer and further improve the cell adhesion properties, and increases the surface of contact between the human body and the spinal implant.

According to preferred embodiments of the current invention, the integration layer has a thickness in the nanometer range. It has been found that such thicknesses confer to the implant better elasticity properties and decreases the risk that vertebrae become damaged. Preferably, the thickness of the integration layer is between 0,3 nm and 5 µm, preferably between 100 nm and 2,5 µm, more preferably about 250nm, or even 250 nm.

According to preferred embodiments of the current invention, the metal containing coating, has a thickness in the nanometer range.

According to preferred embodiments of the current invention, the metal coating has a thickness in the nanometer range.

According to preferred embodiments of the current invention, the biocompatible polymer is one of the list comprising polyether ketone ketone (PEKK), polyether ether ketone (PEEK), ultra high molecular weight polyethylene, preferably polyetheretherketone (PEEK). It has been found that such material have mechanical properties that are adapted to a clinical use, for example as spinal implants.

According to preferred embodiments of the current invention, the thickness of the integration layer remains substantially constant. It has been established that a substantial constant thickness, preferably a constant thickness, of the integration layer allows the texture of the spinal implant not to be altered.

According to preferred embodiments of the current invention, the integration is layer is not porous.

According to preferred embodiments of the current invention, the spinal implant is an intersomatic cage. Examples of intersomatic cages can for example be found in WO2011/054958.

According to preferred embodiments of the current invention, the metal containing coating is located on the vertebrae contacting surfaces, allowing a more direct and/or intimate contact between the integration layer and the human body cells and thus optimizing the tolerance of the human body towards the implant and therefore, the healing.

According to preferred embodiments of the current invention, the implant comprises cavities extending between the vertebrae contacting surfaces and at least part of the metal containing coating is located on the outer surface of the cavities. It has been found that such cavities make a more intimate interconnection between the vertebrae contacting the vertebrae contacting surfaces possible.

The invention also relates to a method for making the spinal implant according to the invention. The method comprises coating a substrate having a texture at the outer surface of said substrate with the coating. It has been found that this method of coating is easily applicable.

According to an example, the coating is done by a technique which does not require substantially heating the substrate, for example comprising PEEK as described above, such as for example physical vapour deposition, preferably a cold vapour deposition technique, preferably a sputtering deposition technique, for example by any one of or combinations of direct current (DC) sputtering, alternating current (AC) sputtering, radio frequency (RF) sputtering, reactive sputtering, for example to make a metal oxide coating. It has been found that this technique allows the production of controlled coating thicknesses, thus generating homogeneous titanium coating onto the polymer material, for example PEEK. The use of such sputter deposition technique allows the thickness of the integration layer to be controlled with a precision in the nanometer range, thus allowing a precise monitoring of the process. Other physical vapour deposition techniques such as plasma deposition although less preferred are also possible.

According to an example, during sputtering an ion-beam, preferably an Argon-beam, preferably a high energy ion-beam, is directed at a surface, preferably a metal containing surface, for example a metal surface, for example a titanium surface, for example a metal containing wafer, for example a metal wafer, for releasing atoms, for example metal atoms, from the surface, and wherein the released atoms are then deposited onto the texture of the substrate.

According to an example, the substrate is being made by moulding, for example injection moulding, with a thereto provided mould as in such embodiments, larger numbers of substrates can be relatively easily made.

According to an example, the mould texture complementing the substrate's texture being provided such as to create the substrate's texture during moulding, for example injection moulding, of the substrate. Such a mould allowing even the serial production of substrates by moulding without having to provide the texture on the substrates in a separate step after moulding.

According to an example, the mould is substantially made of steel, for example stainless steel.

According to further examples the texture is being made in the mould by an etching process, preferably chemically etching. For example the pattern of the texture is being heated by a laser after which the heated area is at least partly being etched away, for example by chemically etching thus creating the mould texture in the mould. Although other techniques, for example optical lithography or electron beam lithography, for applying the texture in the mould are possible, such a technique has been found to be easier to apply to the typically curved surfaces in the mould.

Other details and advantages of the spinal implant and the method according to the invention will become apparent from the enclosed figures and description of preferred embodiments of the invention.

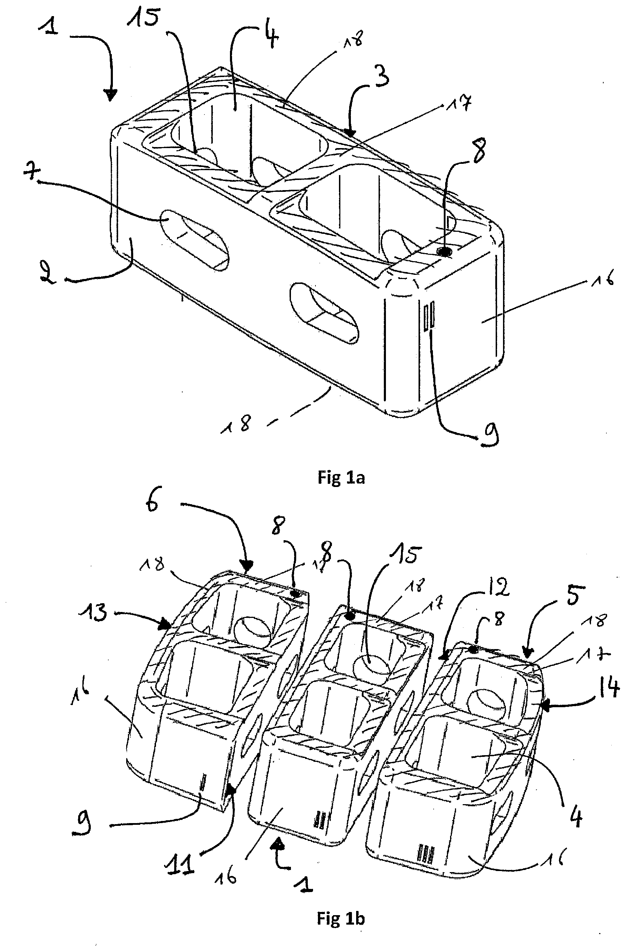

Figure 1a shows a view in perspective of a spinal implant, more in particular an intersomatic cage, more in particular a median intersomatic cage, according to the invention.

Figure 1b shows a view of a kit of spinal implants according to the invention.

The median intersomatic cage 1 shown on figure 1a has a first lateral wall 2 and a second lateral wall 3 positioned on both sides of the bone tissue cavities 4. Both lateral walls 2, 3 preferably, as shown in the figures, are significantly planar. Furthermore, as a result of the conception of the lateral walls 2, 3, this median intersomatic cage 1 can be positioned in the two possible ways between two vertebrae. As shown in figure 1, each lateral wall 2, 3 preferably comprises openings 7 allowing for blood-circulation for osteo-integration, once the median intersomatic cage 1 has been implanted between the two vertebrae. Such openings 7 are however not critical for the invention and can be omitted.

The implant 1 shown in figure 1 is substantially beam-shaped, this is however not critical for the invention and other shapes deemed appropriate by the person skilled in the art can be used.

The median intersomatic cage 1 as shown in figure 1a and 1b comprises two metal indicators, for example made from tantalum although any other material is possible, 8 (one of which is not visible on figure 1a), in order to check with for exmaple X-ray imaging techniques that the median intersomatic cage has not migrated once that it has been implanted into the patient.

The median intersomatic cage 1 as shown in figure 1a and 1b preferably comprises a mechanical engraving 9 allowing its identification with respect to other intersomatic cages. This mechanical engraving 9 is not critical for the invention.

The median intersomatic cage 1 for example is implanted in the space between two vertebrae in a posterior unilateral way, using for example a cage-holder (not represented on figure 1a).

Preferably, the median intersomatic cage 1 has been sterilized using gamma-rays.

The intersomatic cage can be used alone or coupled to other intersomatic cages, according to, for example, the morphology of the patient.

Figure 1b is a perspective view of a kit 10 kit of three spinal implants according to the invention. Although several spinal implants may be necessary to obtain the desired result, this is not critical for the invention as also a single spinal implant may be sufficient or, for example, two, four, five, six seven, eight, or even more spinal implants 1.

The kit 10 specifically shown in figure 1b comprises two intersomatic cages 5, 6 and one median intersomatic cage 1. The exact configuration of the kit 10 is however not critical for the invention.

The intersomatic cages 5, 6 and the median intersomatic cage 1 of kit 10 comprise two cavities 4 each, for bone tissue, as well as openings 7 allowing blood circulation for osteo-integration once said intersomatic cages 5,6 and median intersomatic cage 1 are implanted in an intervertrebrae space, preferably of lumbar vertrebrae. The number, dimensions, shape and presence of the cavities 4 are however not critical for the invention and can be determined by the person skilled in the art depending on the desired configuration.

The intersomatic cages 5, 6 and the median intersomatic cage 1 of kit 10 moreover comprise fixation means 15, allowing the fixation of a cage-holder. The fixation means can be any means that are deemed appropriate by the person skilled in the art and can for example be in the form of holes, for example screw holes for receiving screws. This is however not critical for the invention and other configurations deemed appropriate by the person skilled in the art are possible.

The median intersomatic cage 1 has lateral walls 2, 3 that are significantly planar, and that are conceived to form a contact surface adapted respectively to the intern surface 11 of the intersomatic cage 6 and to the intern surface 12 of the intersomatic cage 5. The intern surfaces 11, 12 are significantly planar as well. The outer surface 13 of the intersomatic cage 6 and the outer surface 14 of the intersomatic cage 5 preferably are significantly curved from the side, as shown in the figures, in order to facilitate the shift of said cages 5, 6 after their introduction between the vertebrae.

Using the kit 10 according to the invention, the surgeon can decide to implant the intersomatic cages 5, 6 with or without the median intersomatic cage 1 in between two vertebrae, according to the patient's morphology. Also, the space between the intersomatic cages are adapted to the morphology of the patient therefore allowing to fit a very broad panel of morphologies, with one single size of implants.

The intersomatic cages 5, 6 and the median intersomatic cage 1 comprise a mechanical engraving 9 allowing their identification. The indication "II" is engraved on cage 1, and the indications "I" and "III" are engraved respectively on cages 5 and 6. These mechanical engravings 9 are not critical for the invention.

Moreover, the intersomatic cages 5, 6 and the median intersomatic cage 1 of kit 10 according to the invention have been sterilized using gamma-rays.

In addition, The intersomatic cages 5, 6 and the median intersomatic cage 1 preferably comprise, as shown in figure 1b, two tantalum indicators 8 (one of which is not visible on figure 1b), in order to check with X-ray imaging techniques that the median intersomatic cage has not migrated once that it has been implanted into the patient.

The implant 1, 5, 6 comprise a substrate 16. The substrate 16 preferably forms a substantial part of the implant, as can be seen in the figures. As can be further seen in the figures the implant further comprises at least one integration layer 17 at an outer surface of the spinal implant. For reasons of clarity the integration 17 has only been shown on top of the substrate 16. This is however not critical for the invention and the integration layer 17 can also be provided on other surfaces such as, for example, the later walls 2, 3, the bottom of the substrate, etc. Preferably, the integration layer 17 is located on the outer surface of the substrate 16 and/or the integration layer 17 is located on vertebrae contacting surfaces 18 and/or the outer surface of the cavities 4.

The substrate 16 of the implant according to the invention, for example the intersomatic cages 5, 6 and of the median intersomatic cage 1 shown in figure 1a and is made of a biocompatible polymer, for example any one ore more of polyether ether ketone (PEEK), polyether ketone ketone (PEKK), ultra-high molecular weight polyethylene (UMHPWE). Preferably, however the substrate is substantially made of a single biocompatible polymer, for example PEEK.

Preferably, a texture is engineered on the PEEK surface in which the distance between adjacent local extrema is less than 1 mm, for example in micrometer range. Then, after coating the integration onto the texture the texture can remain substantially unaffected due to the relatively small thickness of the integration layer.

The integration layer 17 for example is in the form of a metal containing coating applied onto the substrate. The metal containing coating can be in the form of a metal-only coating. In a further embodiment of the invention, the metal containing coating can be a metal-only layer, for example titanium, covered with a layer of metal oxide, for example titanium oxide, which can be a result of the natural oxidation of the metal due to air exposure, or deposited with a sputtering technique, for example the same sputtering technique and/or reactive sputtering. Another embodiment of this invention is a metal-containing coating on a substrate, the metal containing coating being for example exclusively a metal oxide, for example a layer of titanium oxide deposited with a sputtering technique.

The integration layer 17 for example can also be in the form of a calcium phosphate coating, which can for example be sputtered on top of the substrate 16, or can be applied in combination with the metal containing coating. For example, the integration layer 17 can be in the form of a calcium phosphate coating, which is sputtered on top of the previously applied metal containing coating, preferably metal coating, more preferably titanium coating.

## Claims

1. A spinal implant (1, 5, 6) for positioning in between two vertebrae, comprising a substrate (16) and at least one integration layer (17) at an outer surface of the spinal implant for facilitating the acceptance of said implant by the human body, the substrate (16) of the implant being formed of a biocompatible polymer material and comprising at its outer surface the integration layer (17), the spinal implant having a texture engineered in the form of grooves, wherein the integration layer (17) is in the form of a coating applied onto the substrate (16), and wherein the coating is a metal containing coating,
**characterized in that** the integration layer has a thickness in between 0.3 nm - 5 µm and wherein the distance between adjacent local extrema in the spinal implant texture are between 0.3 µm and 150 µm.

2. A spinal implant according to claim 1 wherein the grooves comprise at least a first and a second group of substantially parallel grooves, wherein the grooves of the two groups cross each other in the form of an array of quadrilaterals.

3. A spinal implant according to any one of the preceding claims wherein the depth between adjacent local extrema in the spinal implant texture are between 0.3 µm and 150 µm.

4. A spinal implant according to any one of the preceding claims, wherein the metal of the coating is one of the list consisting of titanium, aluminium, vanadium, tantalum, stainless steel, and alloys of these metals.

5. A spinal implant according to any one of the preceding claims, wherein the metal containing coating comprises a metal coating.

6. A spinal implant according to claim 5, wherein the metal of the metal coating is one of titanium and a titanium alloy, more preferably titanium.

7. A spinal implant according to any one of the preceding claims, wherein the metal containing coating comprises a metal oxide, for example titanium oxide.

8. A spinal implant according to any one of the preceding claims, wherein the integration layer is located on the outer surface of the substrate.

9. A spinal implant according to any one of the preceding claims, wherein the distance, preferably depth, between the adjacent local extrema is between0.5 µm and 130 µm.

10. A spinal implant according to any one of the preceding claims, wherein the thickness of the integration layer is between 100 nm and 2.5 µm, more preferably 250nm.

11. A spinal implant according to any one of the preceding claims, wherein the biocompatible polymer is selected from the list comprising polyether ether ketone (PEEK), polyether ketone ketone (PEKK), ultra high molecular weight polyethylene (UMHPWE).

12. A spinal implant according to any one of the preceding claims, wherein the thickness of the integration layer remains substantially constant.

13. A spinal implant according to any of the preceding claims, wherein the spinal implant is an intersomatic cage.

14. A spinal implant according to any one of the preceding claims, wherein the integration layer is located on the vertebrae contacting surfaces.

15. A method for making the spinal implant according to any one of the preceding claims, comprising coating a substrate having a texture at the outer surface of said substrate with the coating.

## Patentansprüche

1. Ein Wirbelsäulenimplantat (1, 5, 6) zur Positionierung zwischen zwei Wirbeln, welches ein Substrat (16) und zumindest eine Integrationsschicht (17) an einer äußeren Oberfläche des Wirbelsäulenimplantats zur Förderung der Akzeptanz des erwähnten Implantats durch den menschlichen Körper umfasst, wobei das Substrat (16) des Implantats aus einem biokompatiblen Polymermaterial geformt ist und an seiner äußeren Oberfläche die Integrationsschicht (17) umfasst, wobei das Wirbelsäulenimplantat eine Textur hat, technisch ausgeführt in der Form von Rillen, wobei die Integrationsschicht (17) in der Form einer Beschichtung auf das Substrat (16) aufgetragen ist, und wobei die Beschichtung eine metallhaltige Beschichtung ist,
**dadurch gekennzeichnet, dass** die Integrationsschicht eine Dicke von zwischen 0,3 nm und 5 µm hat, und wobei der Abstand zwischen nebeneinander liegenden lokalen Extrema in der Wirbelsäulenimplantattextur zwischen 0,3 µm und 150 µm beträgt.

2. Ein Wirbelsäulenimplantat nach Anspruch 1, wobei die Rillen zumindest eine erste und eine zweite Gruppe von im Wesentlichen parallelen Rillen umfassen, wobei die Rillen der zwei Gruppen einander in der Form eines Bereichs von Vierecken kreuzen.

3. Ein Wirbelsäulenimplantat nach irgendeinem der vorigen Ansprüche, wobei die Tiefe zwischen nebeneinander liegenden lokalen Extrema in der Wirbelsäulenimplantattextur zwischen 0,3 µm und 150 µm beträgt.

4. Ein Wirbelsäulenimplantat nach irgendeinem der vorigen Ansprüche, wobei das Metall der Beschichtung eines aus der Liste bestehend aus Titan, Aluminium, Vanadium, Tantal, Edelstahl, und Legierungen dieser Metalle ist.

5. Ein Wirbelsäulenimplantat nach irgendeinem der vorigen Ansprüche, wobei die metallhaltige Beschichtung eine Metallbeschichtung umfasst.

6. Ein Wirbelsäulenimplantat nach Anspruch 5, wobei das Metall der Metallbeschichtung eines von Titan und einer Titanlegierung ist, besser Titan.

7. Ein Wirbelsäulenimplantat nach irgendeinem der vorigen Ansprüche, wobei die metallhaltige Beschichtung ein Metalloxid umfasst, zum Beispiel Titanoxid.

8. Ein Wirbelsäulenimplantat nach irgendeinem der vorigen Ansprüche, wobei sich die Integrationsschicht auf der äußeren Oberfläche des Substrates befindet.

9. Ein Wirbelsäulenimplantat nach irgendeinem der vorigen Ansprüche, wobei der Abstand, bevorzugt die Tiefe, zwischen den nebeneinander liegenden lokalen Extrema zwischen 0,5 µm und 130 µm beträgt.

10. Ein Wirbelsäulenimplantat nach irgendeinem der vorigen Ansprüche, wobei die Dicke der Integrationsschicht zwischen 100 nm und 2,5 µm beträgt, besser 250 nm.

11. Ein Wirbelsäulenimplantat nach irgendeinem der vorigen Ansprüche, wobei das biokompatible Polymer ausgewählt wird aus der Liste, die Polyetheretherketon (PEEK), Polyetherketonketon (PEKK), Polyethylen mit ultrahoher Molekülmasse (UMHPWE) umfasst.

12. Ein Wirbelsäulenimplantat nach irgendeinem der vorigen Ansprüche, wobei die Dicke der Integrationsschicht im Wesentlichen konstant bleibt.

13. Ein Wirbelsäulenimplantat nach irgendeinem der vorigen Ansprüche, wobei das Wirbelsäulenimplantat ein zwischenwirbel-käfig ist.

14. Ein Wirbelsäulenimplantat nach irgendeinem der vorigen Ansprüche, wobei sich die Integrationsschicht auf den Wirbelkontaktflächen befindet.

15. Ein Verfahren zur Herstellung des Wirbelsäulenimplantats nach irgendeinem der vorigen Ansprüche, welches das Beschichten eines Substrates umfasst, das eine Textur auf der äußeren Oberfläche des erwähnten Substrates mit der Beschichtung hat.

## Revendications

1. Implant spinal (1, 5, 6) à positionner entre deux vertèbres, comprenant un substrat (16) et au moins une couche d'intégration (17) au niveau d'une surface extérieure de l'implant spinal pour faciliter l'acceptation dudit implant par le corps humain, le substrat (16) de l'implant étant formé d'une matière polymère compatible d'un point de vue biologique et comprenant au niveau de sa surface extérieure la couche d'intégration (17), l'implant spinal ayant une texture ingéniérisée sous la forme de rainures, dans lequel la couche d'intégration (17) est sous la forme d'un revêtement appliqué sur le substrat (16), et dans lequel le revêtement est un revêtement contenant un métal,
**caractérisé en ce que** la couche d'intégration a une épaisseur entre 0,3 nm - 5 µm et dans lequel la distance entre extrêmes locaux adjacents dans la texture d'implant spinale est entre 0,3 µm et 150 µm.

2. Implant spinal selon la revendication 1, dans lequel les rainures comprennent au moins un premier et un second groupe de rainures sensiblement parallèles, dans lequel les rainures des deux groupes se croisent sous la forme d'un réseau de quadrilatères.

3. Implant spinal selon l'une quelconque des revendications précédentes, dans lequel la profondeur entre des extrêmes locaux adjacents dans la texture d'implant spinal est entre 0,3 µm et 150 µm.

4. Implant spinal selon l'une quelconque des revendications précédentes, dans lequel le métal du revêtement est l'un de la liste consistant en du titane, de l'aluminium, du vanadium, du tantale, de l'acier inoxydable et des alliages de ces métaux.

5. Implant spinal selon l'une quelconque des revendications précédentes, dans lequel le revêtement contenant un métal comprend un revêtement de métal.

6. Implant spinal selon la revendication 5, dans lequel le métal du revêtement de métal est l'un du titane et d'un alliage de titane, plus préférentiellement du titane.

7. Implant spinal selon l'une quelconque des revendications précédentes, dans lequel le revêtement contenant un métal comprend un oxyde de métal, par exemple de l'oxyde de titane.

8. Implant spinal selon l'une quelconque des revendications précédentes, dans lequel la couche d'intégration est située sur la surface extérieure du substrat.

9. Implant spinal selon l'une quelconque des revendications précédentes, dans lequel la distance, de préférence la profondeur, entre les extrêmes locaux adjacents est entre 0,5 µm et 130 µm.

10. Implant spinal selon l'une quelconque des revendications précédentes, dans lequel l'épaisseur de la couche d'intégration est entre 100 nm et 2,5 µm, plus préférentiellement 250 nm.

11. Implant spinal selon l'une quelconque des revendications précédentes, dans lequel le polymère compatible d'un point de vue biologique est sélectionné à partir de la liste comprenant du polyétheréthercétone (PEEK), du polyéther cétone cétone (PEKK), du polyéthylène de masse moléculaire ultra-élevée (UMHPWE).

12. Implant spinal selon l'une quelconque des revendications précédentes, dans lequel l'épaisseur de la couche d'intégration reste sensiblement constante.

13. Implant spinal selon l'une quelconque des revendications précédentes, dans lequel l'implant spinal est une cage intersomatique.

14. Implant spinal selon l'une quelconque des revendications précédentes, dans lequel la couche d'intégration est située sur les surfaces entrant en contact avec les vertèbres.

15. Procédé pour fabriquer l'implant spinal selon l'une quelconque des revendications précédentes, comprenant le revêtement d'un substrat ayant une texture au niveau de la surface extérieure dudit substrat avec le revêtement.
